# EUROPÄISCHE PATENTSCHRIFT

(11) **EP 1 758 504 B1**
(45) Veröffentlichungstag und Bekanntmachung des Hinweises auf die Patenterteilung: **30.07.2008**
(21) Anmeldenummer: 05716574.8
(22) Anmeldetag: 12.04.2005
(51) Int. Cl.: A61B 8/00, A61B 19/00

(54) **VERFAHREN UND VORRICHTUNG ZUR EINGRIFFSLOSEN BESTIMMUNG MARKANTER STRUKTUREN DES MENSCHLICHEN ODER TIERISCHEN KÖRPERS**
METHOD AND APPARATUS FOR THE NON-INVASIVE DETERMINATION OF PROMINENT STRUCTURES OF THE HUMAN OR ANIMAL BODY
PROCEDE ET DISPOSITIF DE DETERMINATION NON INVASIVE DE STRUCTURES PROEMINENTES DU CORPS D'UN ETRE HUMAIN OU D'UN ANIMAL

(30) Priorität: 25.05.2004 DE 102004026525
(43) Veröffentlichungstag der Anmeldung: 07.03.2007
(73) Patentinhaber: AESCULAP AG & Co. KG, 78532 Tuttlingen (DE); Universität Ulm, 89073 Ulm (DE)
(72) Erfinder: KOZAK, Josef, 78532 Tuttlingen (DE); KEPPLER, Peter, 89075 Ulm (DE)
(74) Vertreter: Boehme, Ulrich
(86) Internationale Anmeldenummer: PCT/EP2005/003847
(87) Internationale Veröffentlichungsnummer: WO 2005/115246

(56) Entgegenhaltungen:
- WO-A-20/04001569
- US-A1- 2002 147 455
- KEPPLER P ET AL: "Die sonographische Bestimmung der Beingeometrie" DER ORTHOPÄDE, Bd. 28, Nr. 12, Dezember 1999 (1999-12), Seiten 1015-1022, XP002331181 ISSN: 0085-4530
- STRECKER W ET AL: "Computertomographische Torsionswinkelbestimmung der unteren Extremitäten" UNFALLCHIRURG, Bd. 97, Nr. 11, 1994, Seiten 609-613, XP008048047 ISSN: 0177-5537

## Beschreibung

Die Erfindung betrifft ein Verfahren zur eingriffslosen Bestimmung der Lage und Orientierung markanter Strukturen im Inneren eines menschlichen oder tierischen Körpers, bei dem man den Körper im Bereich der markanten Strukturen mittels eines Ultraschall aussendenden und Ultraschall empfangenden Ultraschallkopfes mit Ultraschallstrahlung bestrahlt, die an der markanten Struktur reflektierte Ultraschallstrahlung empfängt und eine der Laufzeit der reflektierten Strahlung entsprechende Abbildung auf einer Anzeige darstellt, wobei man die Lage und Orientierung des Ultraschallkopfes und des Körpers im Bereich der markanten Struktur mit Hilfe von am Ultraschallkopf und am Körper festgelegten Markierelementen mittels eines Navigationssystems bestimmt und wobei man auf der Anzeige eine markante Struktur auswählt und aufgrund der Strahlrichtung des Ultraschallkopfes und der gemessenen Laufzeit der Ultraschallstrahlung die Lage und Orientierung der markanten Struktur relativ zum Ultraschallkopf und aus der Lage und Orientierung des Ultraschallkopfes relativ zum Körper die Lage und Orientierung der markanten Struktur relativ zum Körper bestimmt.

Die Erfindung betrifft weiterhin eine Vorrichtung zur Durchführung eines derartigen Verfahrens nach einem der Ansprüche 1 oder 2 mit einem Ultraschallkopf zur Aussendung und zum Empfang von Ultraschallstrahlung und mit einer Anzeige zur Darstellung einer der Laufzeit einer ausgesandten, an einer markanten Struktur des Körpers reflektierten Ultraschallstrahlung entsprechenden Abbildung, wobei Markierelemente für den Ultraschallkopf und den Körper vorgesehen sind, die Teil eines Navigationssystems sind, und mit einer Datenverarbeitungsanlage.

Um bestimmte markante Strukturen im Inneren eines Körpers zu bestimmen, beispielsweise bestimmte Knochenstrukturen, und deren genaue Lage und Orientierung relativ zum Körper festzustellen, ist es bisher notwendig, den Körper zu öffnen und im Inneren des Körpers diese Struktur mit einem Tastinstrument zu erfassen, dessen Lage und Orientierung relativ zum Körper beispielsweise über ein Navigationssystem bestimmt werden kann. Dadurch erhält man Auskunft über die Lage und gegebenenfalls Orientierung der markanten Strukturen relativ zum Körper.

Nachteilig ist bei diesem Verfahren, daß dazu eine Öffnung des Körpers notwendig ist, wenn man derartige Strukturen durch Weichgewebe hindurch ertastet, können sich Ungenauigkeiten einstellen.

Andererseits ist es bekannt, Körperstrukturen im Inneren des Körpers über Ultraschallstrahlung abzutasten. Bei bekannten Ultraschall-Abtastverfahren wird über einen Ultraschallkopf Ultraschallstrahlung in den Körper gerichtet, wird dort an markanten Strukturen reflektiert, und vom Ultraschallkopf wird die reflektierte Strahlung wieder aufgenommen. Aus der Laufzeit läßt sich der Abstand der Reflexionsflächen von dem Ultraschallkopf bestimmen, und aus der Strahlrichtung des Ultraschallkopfes auch die Lage der markanten Strukturen. Insgesamt kann man auf diese Weise zwar die Form der markanten Strukturen erfassen und abbilden, erhält aber nicht genaue Angaben über Lage und Orientierung relativ zum Körper.

Es ist weiterhin bekannt, die Beingeometrie sonographisch zu bestimmen, und zwar unter Verwendung eines navigierten Ultraschallkopfes, also eines Ultraschallkopfes, dessen Position und Orientierung im Raum ebenso feststellbar ist wie die Position und Orientierung von Körperteilen (Keppler P. et al: "Die sonographische Bestimmung der Beingeometrie" DER ORTHOPÄDE, Bd. 28, Nr. 12, Dezember 1999, Seiten 1015-1022, XP002331181 ISSN: 0085-4530). Mit diesem bekannten Verfahren sind die Längen und die Torsionen im Bereich der unteren Extremität bestimmt worden.

Es ist Aufgabe der Erfindung, ein Verfahren der gattungsgemäßen Art so auszubilden, daß ohne Eingriff in den Körper eine charakteristische Ebene des Hüftknochens bestimmt werden kann.

Diese Aufgabe wird bei einem Verfahren der eingangs beschriebenen Art erfindungsgemäß dadurch gelöst, daß man als markante Struktur die Beckenkämme eines Hüftknochens auf der Anzeige mit einer vorgegebenen geometrischen Struktur möglichst gut zur Deckung bringt und aus der Lage der vorgegebenen geometrischen Struktur auf der Anzeige Strukturdaten der markanten Struktur des Körpers bestimmt, wobei die vorgegebene geometrische Struktur ein Kreis ist und man als Strukturdaten der vorgegebenen geometrischen Struktur eine Kugel bestimmt mit dem Kreis der vorgegebenen Struktur als Großkreis, und daß man eine charakteristische Ebene eines Hüftknochens derart wählt, daß in ihr ein charakteristischer Punkt der Symphyse liegt und daß die charakteristische Ebene an der Oberfläche der beiden Kugeln anliegt, die den Beckenkämmen zugeordnet sind. Die auf diese Weise bestimmte Ebene kann man als charakteristische Ebene des Hüftknochens bezeichnen, diese Ebene kann beispielsweise als Grundlage für die geometrische Beschreibung des Hüftknochens und die Wahl eines hüftknochenfesten Koordinatensystems dienen.

Der Erfindung liegt auch die Aufgabe zugrunde, eine Vorrichtung zur Durchführung dieses Verfahrens vorzuschlagen.

Diese Aufgabe wird erfindungsgemäß durch eine Vorrichtung mit den Merkmalen des Patentanspruches 3 gelöst.

Dabei ist es vorteilhaft, wenn der Ultraschallkopf die Ultraschallstrahlung über eine Vielzahl von Sende- und Empfangseinrichtungen aussendet beziehungsweise empfängt, die in eine Reihe angeordnet sind und die Ultraschallstrahlung in eine gemeinsame Ebene abstrahlen.

Die nachfolgende Beschreibung bevorzugter Ausführungsformen der Erfindung dient im Zusammenhang mit der Zeichnung der näheren Erläuterung. Es zeigen:
- Figur 1 :: eine schematische Ansicht eines navigierten mechanischen Tasters zur Abtastung markanter Strukturen im Körper entsprechend dem Stand der Technik;
- Figur 2 :: eine schematische Ansicht eines navigierten Ultraschallkopfes in Anlage an einem Beckenkamm des Hüftknochens mit zugehöriger Darstellung der vom Ultraschallkopf gelieferten Abbildung auf einer Anzeige;
- Figur 3 :: eine Ansicht ähnlich Figur 2 mit dem Ultraschallkopf in Anlage am anderen Beckenkamm;
- Figur 4 :: eine Ansicht ähnlich Figur 2 mit dem Ultraschallkopf in Anlage an das Symphyse des Hüftknochens;
- Figur 5 :: eine schematische Darstellung zur Auswertung der Abbildungen der Figuren 2 bis 4 und zur Bestimmung einer für den Hüftknochen charakteristischen Ebene;
- Figur 6 :: eine Ansicht ähnlich Figur 2 mit der Anlage des Ultraschallkopfes an einem Oberschenkelknochen und
- Figur 7 :: eine Ansicht ähnlich Figur 2 mit einem an das Fußgelenk angelegten Ultraschallkopf.

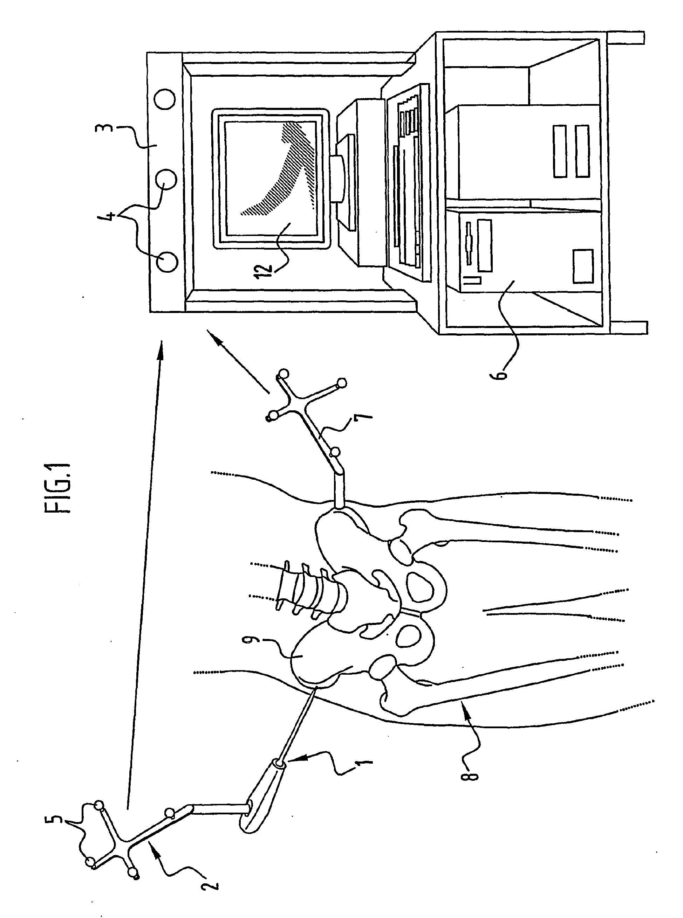

In Figur 1 ist eine herkömmliche Vorrichtung zur mechanischen Abtastung einer markanten Struktur des Körpers dargestellt. Diese Einreichung umfaßt einen mechanischen Taster 1, an dem ein Markierelement 2 eines herkömmlichen Navigationssystems 3 festgelegt ist, dieses Navigationssystem 3 umfaßt mehrere Sende- und Empfangseinheiten 4, die elektromagnetische Strahlung aussenden, die an Reflektoren 5 des Markierelementes 2 reflektiert und von den Sende- und Empfangseinheiten 4 wieder aufgefangen wird. Aus diesen Signalen kann das Navigationssystem 3 in an sich bekannter Weise die genaue Lage und Orientierung des Markierelementes 2 und damit des Tasters 1 im Raum feststellen. Die entsprechenden Positionsdaten werden einer Datenverarbeitungsanlage 6 zugeführt.

Ein weiteres Markierelement 7, das gleich aufgebaut ist wie das Markierelement 2, ist an einem Teil des Körpers 8 befestigt, dessen markante Struktur bestimmt werden soll. Im dargestellten Ausführungsbeispiel ist das Markierelement 7 an einem Hüftknochen 9 festgelegt, beispielsweise mittels einer in den Hüftknochen 9 eingeschraubten Knochenschraube. Derartige Markierelemente werden zu Operationen üblicherweise verwendet und dienen dann der Bestimmung der Lage und Orientierung des entsprechenden Körperteils während einer Operation.

Es ist aber auch ohne weiteres möglich, derartige Markierelemente am ungeöffneten Körper anzuordnen, beispielsweise mit Hilfe von Haltern, die an der Außenseite des Körpers durch die Weichgewebeteile hindurch festgelegt werden, eine solche Festlegung kann über Bänder erfolgen etc.

Das Navigationssystem 3 kann auf diese Weise sowohl Lage und Orientierung des Tasters 1 als auch Lage und Orientierung des Körperteils bestimmen, an dem eine Struktur lokalisiert werden soll. Diese Lokalisierung erfolgt bei der in Figur 1 beschriebenen Vorrichtung, die dem bekannten Stand der Technik entspricht, dadurch, daß die Spitze des Tasters 1 an die entsprechende Struktur angelegt wird, dazu muß der Körper geöffnet sein, die entsprechende Struktur muß freigelegt werden.

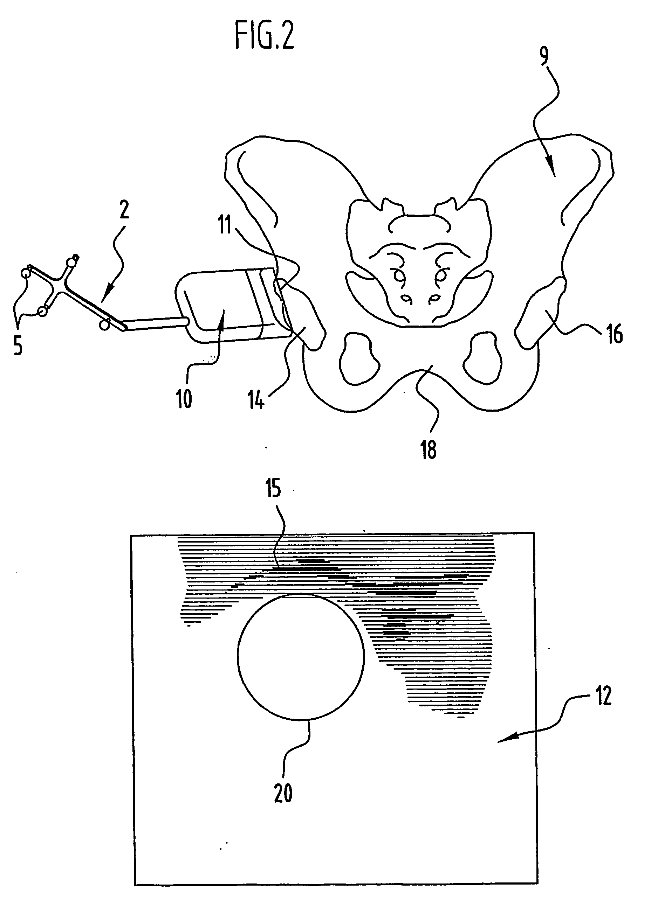

Um dies zu vermeiden, wird bei einem Verfahren, wie es nachstehend anhand der Figuren 2 bis 7 erläutert wird, statt eines mechanischen Tasters ein Ultraschallkopf 10 verwendet, der an sich zur Untersuchung körperinnerer Strukturen bekannt ist und der mittels einer Anlagefläche 11 an der Außenseite des Körpers anlegbar ist. Ein solcher Ultraschallkopf 10 strahlt beispielsweise über mehrere Sende- und Empfangseinheiten, die in einer Reihe angeordnet sind, Ultraschallstrahlung in eine allen Sende- und Empfangseinheiten gemeinsame Ebene ab und empfängt an inneren Körperstrukturen reflektierte Ultraschallstrahlung, die in dieser Ebene zurückgeworfen wird. Aus der Laufzeit zwischen Aussenden und Empfangen der Strahlung läßt sich der Abstand der reflektierenden Fläche an der inneren Struktur des Körpers zum Ultraschallkopf 10 bestimmen, und diese Daten werden der Datenverarbeitungsanlage 6 zugeführt.

Am Ultraschallkopf 10 ist das Markierelement 2 befestigt, so daß die Lage des Ultraschallkopfes und dessen Orientierung durch das Navigationssystem 3 bestimmt werden.

Das vom Ultraschallkopf 10 erzeugte Muster wird auf einem Bildschirm 12 dargestellt, so daß der Operateur am Bildschirm 12 das Reliefmuster der Ultraschallstrahlung, also der Laufzeitmessungen der Ultraschallstrahlung, ablesen kann. Dieses Muster zeigt bestimmte Strukturen, die sich aus der Struktur der die Ultraschallstrahlung reflektierenden Fläche ergeben, also beispielsweise einer Knochenoberfläche.

Am Beispiel eines Hüftknochens 9 wird anhand der Figuren 2 bis 4 erörtert, wie auf diese Weise bestimmte Strukturen des Hüftknochens bestimmt werden können und wie diese Strukturen zur Charakterisierung des Hüftknochens Verwendung finden können.

Der Ultraschallkopf 10 wird zunächst an einer Seite des Hüftknochens 9 so auf den Körper 8 aufgesetzt, daß er im wesentlichen mit dem Beckenkamm 14 ausgerichtet ist. Die genaue Ausrichtung kann der Operateur anhand der Anzeige auf dem Bildschirm 12 vornehmen, da sich bei exakter Ausrichtung zum Beckenkamm 14 eine besonders scharfe und deutlich erkennbare Struktur ergibt, dadurch läßt sich der Ultraschallkopf weitgehend reproduzierbar an Hüftknochen 9 unterschiedlicher Patienten anlegen.

Dabei ergibt sich eine für diesen Beckenkamm 14 des Hüftknochens 9 charakteristische Abbildung auf dem Bildschirm 12, bei dieser Abbildung sind bogenförmige Bereiche 15 deutlich erkennbar.

In gleicher Weise wird am gegenüberliegenden Beckenkamm 16 vorgegangen. Man erhält dabei eine im wesentlichen gleiche Darstellung am Bildschirm 12 mit bogenförmigen Bereichen 17.

In einem dritten Schritt wird der Ultraschallkopf in der Mitte des Hüftknochens 9 an dessen unterem Ende im Bereich der Symphyse 18 angelegt, dabei erhält man auf dem Bildschirm 12 eine deutliche nach unten weisende Spitze 19, welche die Symphysenstruktur widerspiegelt.

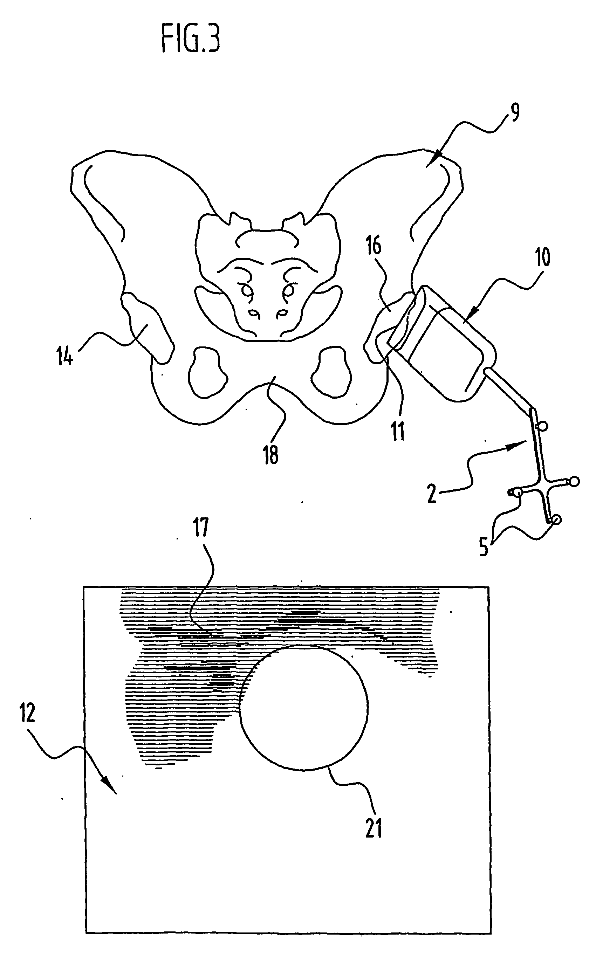

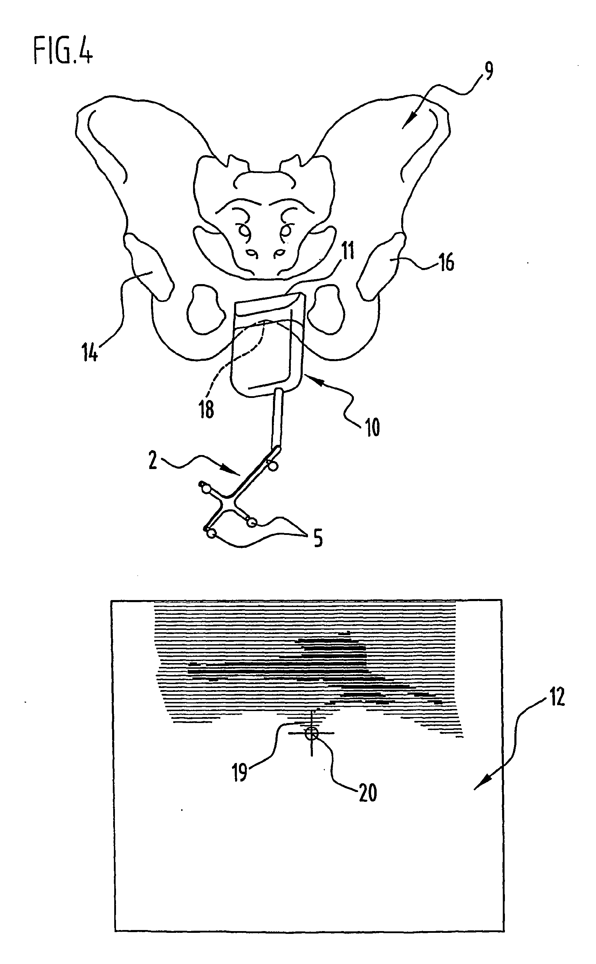

Um aus den abgebildeten Strukturen die notwendigen Positions- und Orientierungsdaten zu erhalten, können auf dem Bildschirm 12 Symbole mit vorgegebener geometrischer Struktur dargestellt und verschoben werden, beispielsweise Kreise 20, 21 (Figuren 2 und 3) und Punkte 22 (Figur 4). Diese Symbole kann der Operateur so auf dem Bildschirm 12 verschieben, daß mit den dargestellten Ultraschallbildern eine bewußte Koordinierung erfolgt, beispielsweise können die Kreise 20 und 21 optimal an die bogenförmigen Bereiche 15 beziehungsweise 17 angelegt werden, der Punkt 22 kann auf die Spitze 19 aufgelegt werden. Damit lassen sich geometrische Datensätze bestimmen, die der Datenverarbeitungsanlage 6 zugeführt werden und die jeweils charakteristisch sind für die mit dem Ultraschallkopf bestrahlten Körperbereiche und markanten Strukturen.

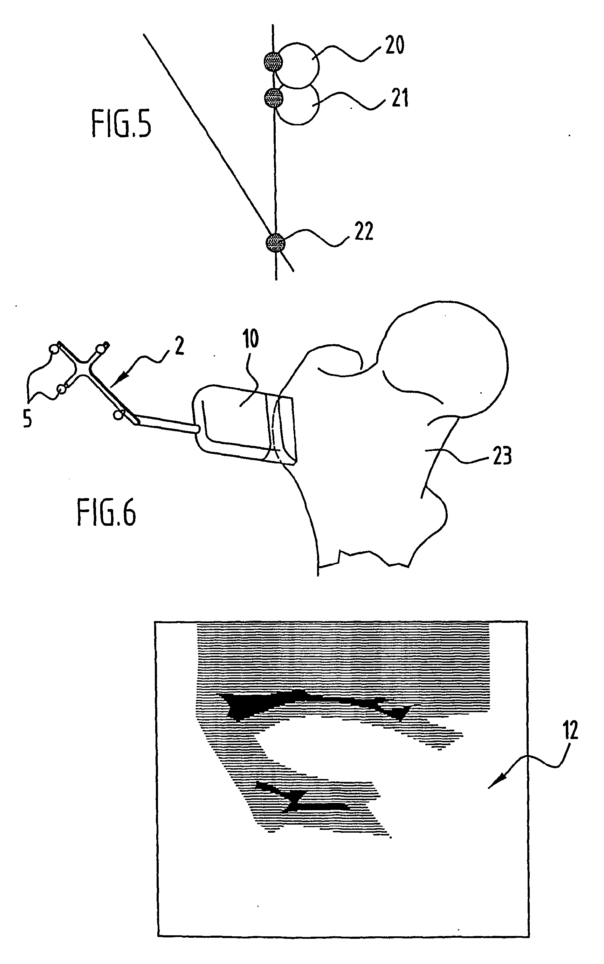

Die Datenverarbeitungsanlage legt durch die beiden Kreise 20, 21 eine Kugel, die denselben Mittelpunkt und denselben Radius aufweist wie die Kreise, so daß die Kreise Großkreise bilden. Dann berechnet die Datenverarbeitungsanlage aus der Lage des Punktes 22 einerseits und den auf diese Weise bestimmten Kugeln andererseits eine Ebene, in der der Punkt 22 liegt und die sich tangential an die beiden Kugeln anlegt (Figur 5). In dieser Ebene liegt ein Punkt jedes Beckenkammes 14, da die Kreise 20, 21 an die bogenförmigen Bereiche 15, 17 des Beckenkammes angelegt waren, so daß dies auch für die Kugeln zutrifft. Es wird also eine Ebene bestimmt, die je einen Punkt jedes Beckenkammes und den an der Symphyse bestimmten Punkt enthält, eine solche Ebene ist eine charakteristische Ebene für die Lage und Orientierung des Hüftknochens, und diese Ebene kann verwendet werden, um in einem weiteren Operationsverlauf ein hüftknocheneigenes Koordinatensystem zu bestimmen und relativ zum Hüftknochen notwendige Positionierungen von Implantaten, Bearbeitungswerkzeugen etc. vorzunehmen.

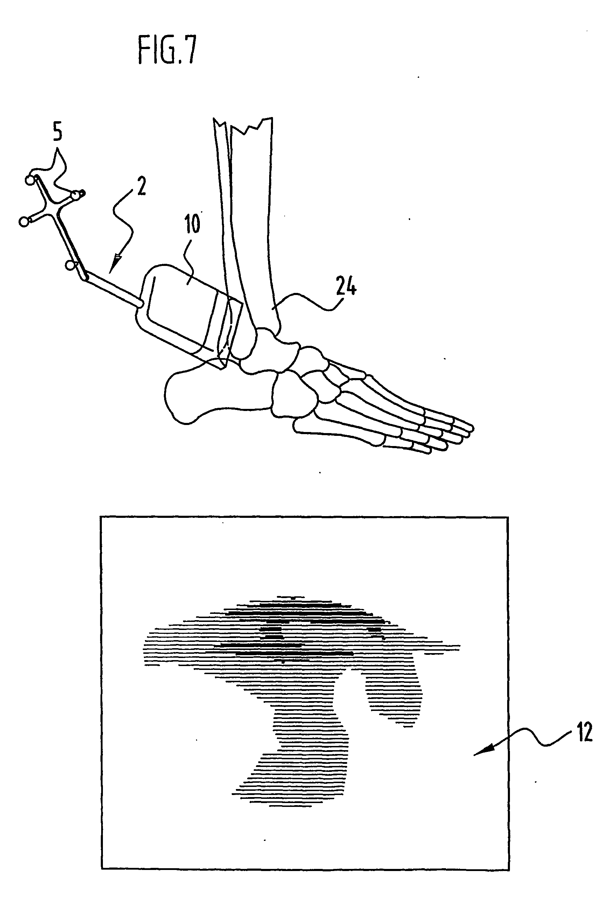

Vorstehend ist die berührungslose Bestimmung charakteristischer Körperstrukturen am Beispiel des Hüftknochens erörtert worden, in ähnlicher Weise lassen sich markante Strukturen an anderen Körperstellen auffinden, beispielsweise am hüftsgelenksseitigen Ende eines Femurknochens 23 (Figur 6), an den der Ultraschallkopf 10 in charakteristischer Weise angelegt wird, oder am knöchelseitigen Ende eines Tibiaknochens 24 (Figur 7). Diese Darstellungen sind lediglich als Anwendungsbeispiele zu sehen. Es versteht sich, daß der Ultraschallkopf 10 an der Körperaußenseite angelegt wird, eine Eröffnung des Körpers also nicht notwendig ist, um die körperinneren Strukturen abzutasten.

## Patentansprüche

1. Verfahren zur eingriffslosen Bestimmung der Lage und Orientierung markanter Strukturen im Inneren eines menschlichen oder tierischen Körpers, bei dem man den Körper im Bereich der markanten Strukturen mittels eines Ultraschall aussendenden und Ultraschall empfangenden Ultraschallkopfes mit Ultraschallstrahlung bestrahlt, die an der markanten Struktur reflektierte Ultraschallstrahlung empfängt und eine der Laufzeit der reflektierten Strahlung entsprechende Abbildung auf einer Anzeige darstellt, wobei man die Lage und Orientierung des Ultraschallkopfes und des Körpers im Bereich der markanten Struktur mit Hilfe von am Ultraschallkopf und am Körper festgelegten Markierelementen mittels eines Navigationssystems bestimmt und wobei man auf der Anzeige eine markante Struktur auswählt und aufgrund der Strahlrichtung des Ultraschallkopfes und der gemessenen Laufzeit der Ultraschallstrahlung die Lage und Orientierung der markanten Struktur relativ zum Ultraschallkopf und aus der Lage und Orientierung des Ultraschallkopfes relativ zum Körper die Lage und Orientierung der markanten Struktur relativ zum Körper bestimmt, **dadurch gekennzeichnet, daß** man als markante Struktur die Beckenkämme eines Hüftknochens auf der Anzeige mit einer vorgegebenen geometrischen Struktur möglichst gut zur Deckung bringt und aus der Lage der vorgegebenen geometrischen Struktur auf der Anzeige Strukturdaten der markanten Struktur des Körpers bestimmt, wobei die vorgegebene geometrische Struktur ein Kreis ist und man als Strukturdaten der vorgegebenen geometrischen Struktur eine Kugel bestimmt mit dem Kreis der vorgegebenen Struktur als Großkreis, und daß man eine charakteristische Ebene eines Hüftknochens derart wählt, daß in ihr ein charakteristischer Punkt der Symphyse liegt und daß die charakteristische Ebene an der Oberfläche der beiden Kugeln anliegt, die den Beckenkämmen zugeordnet sind.

2. Verfahren nach Anspruch 1, **dadurch gekennzeichnet, daß** man einen Ultraschallkopf verwendet, der Ultraschallstrahlung über eine Vielzahl von Sende- und Empfangseinrichtungen aussendet beziehungsweise empfängt, die in einer Reihe angeordnet sind und die Ultraschallstrahlung in eine gemeinsame Ebene abstrahlen.

3. Vorrichtung zur Durchführung des Verfahrens nach einem der Ansprüche 1 oder 2 mit einem Ultraschallkopf (10) zur Aussendung und zum Empfang von Ultraschallstrahlung und mit einer Anzeige (12) zur Darstellung einer der Laufzeit einer ausgesandten, an einer markanten Struktur (14, 16, 18) des Körpers (8) reflektierten Ultraschallstrahlung entsprechenden Abbildung, wobei Markierelemente (2, 7) für den Ultraschallkopf (10) und den Körper (8) vorgesehen sind, die Teil eines Navigationssystems (3) sind, und mit einer Datenverarbeitungsanlage (6), die so programmiert ist, daß sie aus der Relativposition und der Relativorientierung des Ultraschallkopfes (10) zum Körper (8) und der Strahlrichtung des Ultraschallkopfes (10) und der gemessenen Laufzeit der Ultraschallstrahlung die Lage und Orientierung einer auf der Anzeige (12) dargestellten markanten Struktur (14, 16, 18) relativ zum Körper bestimmt, und **dadurch gekennzeichnet, daß** die Datenverarbeitungsanlage (6) so programmiert ist dass sie aus der Lage einer vorgegebenen geometrischen Struktur, die mit den Beckenkämmen eines Hüftknochens auf der Anzeige möglichst gut zur Deckung gebracht worden ist, Strukturdaten der markanten Struktur des Körpers bestimmt, wobei die vorgegebene geometrische Struktur zwei Kreise umfaßt und die Datenverarbeitungsanlage (6) als Strukturdaten der vorgegebenen geometrischen Struktur Kugeln bestimmt mit den Kreisen der vorgegebenen Struktur als Großkreis und eine charakteristische Ebene eines Hüftknochens derart wählt, daß in ihr ein charakteristischer Punkt der Symphyse liegt und die charakteristische Ebene an der Oberfläche der beiden Kugeln anliegt, die den Beckenkämmen zugeordnet sind.

4. Vorrichtung nach Anspruch 3, **dadurch gekennzeichnet, daß** der Ultraschallkopf (10) über eine Vielzahl von Sende- und Empfangseinrichtungen Ultraschallstrahlung aussendet beziehungsweise empfängt, die in einer Reihe angeordnet sind und die Ultraschallstrahlung in eine gemeinsame Ebene abstrahlen.

## Claims

1. Method for the non-invasive determination of the position and orientation of prominent structures inside the body of a human being or an animal, wherein the body is irradiated with ultrasonic radiation in the area of the prominent structures by means of an ultrasonic head which emits ultrasound and receives ultrasound, the ultrasonic radiation reflected at the prominent structure is received, and an image corresponding to the travel time of the reflected radiation is represented on a display, wherein the position and orientation of the ultrasonic head and of the body in the area of the prominent structure are determined by means of a navigation system with the assistance of marking elements fixed to the ultrasonic head and to the body, and wherein a prominent structure is selected on the display, and the position and orientation of the prominent structure relative to the ultrasonic head are determined on the basis of the radiating direction of the ultrasonic head and the measured travel time of the ultrasonic radiation, and the position and orientation of the prominent structure relative to the body are determined from the position and orientation of the ultrasonic head relative to the body, **characterized in that** the iliac crests of a hip bone as prominent structure are brought into as close congruence as possible with a predefined geometrical structure on the display, and structural data relating to the prominent structure of the body are determined from the position of the predefined geometrical structure on the display, the predefined geometrical structure being a circle, and a sphere being determined as structural data of the predefined geometrical structure with the circle of the predefined structure as great circle, and **in that** a characteristic plane of a hip bone is selected such that a characteristic point of the symphysis lies in it, and that the characteristic plane contacts the surface of the two spheres associated with the iliac crests.

2. Method in accordance with claim 1, **characterized in that** an ultrasonic head is used, which emits and receives ultrasonic radiation via a multiplicity of transmitting and receiving devices, which are arranged in a row and radiate the ultrasonic radiation into a common plane.

3. Apparatus for performing the method in accordance with claim 1 or 2, comprising an ultrasonic head (10) for emitting and receiving ultrasonic radiation, and a display device (12) for representing an image corresponding to the travel time of emitted ultrasonic radiation reflected at a prominent structure (14, 16, 18) of the body (8), marking elements (2, 7) which are part of a navigation system (3) being provided for the ultrasonic head (10) and the body (8), and comprising a data processor (6), which is programmed to determine from the relative position and the relative orientation of the ultrasonic head (10) in relation to the body (8) and from the radiating direction of the ultrasonic head (10) and from the measured travel time of the ultrasonic radiation the position and orientation of a prominent structure (14, 16, 18) represented on the display device (12) relative to the body, **characterized in that** the data processor (6) is programmed to determine structural data relating to the prominent structure of the body from the position of a predefined geometrical structure which has been brought into as close congruence as possible with the iliac crests of a hip bone on the display, the predefined geometrical structure comprising two circles, and the data processor (6) determining spheres as structural data of the predefined geometrical structure with the circles of the predefined structure as great circle, and selecting a characteristic plane of a hip bone such that a characteristic point of the symphysis lies in it, and that the characteristic plane contacts the surface of the two spheres associated with the iliac crests.

4. Apparatus in accordance with claim 3, **characterized in that** the ultrasonic head (10) emits and receives ultrasonic radiation via a multiplicity of transmitting and receiving devices, which are arranged in a row and radiate the ultrasonic radiation into a common plane.

## Revendications

1. Procédé pour la détermination non invasive de la position et de l'orientation de structures marquantes à l'intérieur du corps d'un être humain ou d'un animal, selon lequel on irradie le corps dans la zone des structures marquantes avec un rayonnement ultrasonore au moyen d'une tête à ultrasons à émission d'ultrasons et réception d'ultrasons, on effectue la réception du rayonnement ultrasonore réfléchi par la structure marquante, et on représente sur un dispositif de visualisation une image correspondant à la durée de propagation du rayonnement réfléchi, et on détermine par ailleurs, au moyen d'un système de navigation, la position et l'orientation de la tête à ultrasons et du corps dans la région de la structure marquante à l'aide d'éléments de marquage fixés à la tête à ultrasons et au corps, puis on sélectionne une structure marquante sur le dispositif de visualisation, et on détermine, sur la base de la direction du rayonnement de la tête à ultrasons et de la durée de propagation mesurée du rayonnement ultrasonore, la position et l'orientation de la structure marquante par rapport à la tête à ultrasons, et à partir de la position et de l'orientation de la tête à ultrasons par rapport au corps, la position et l'orientation de la structure marquante par rapport au corps,
**caractérisé en ce qu'**en guise de structure marquante on amène les crêtes iliaques d'un os de la hanche le mieux en coïncidence possible avec une structure géométrique prédéterminée sur le dispositif de visualisation, et, à partir de la position de la structure géométrique prédéterminée sur le dispositif de visualisation, on détermine des données de structure de la structure marquante du corps, la structure géométrique prédéterminée étant un cercle, et on détermine en tant que données de structure de la structure géométrique prédéterminée une sphère avec le cercle de la structure prédéterminée en tant que grand cercle, et **en ce qu'**on sélectionne un plan caractéristique de l'os de la hanche de façon telle qu'un point caractéristique de la symphyse y soit situé et que le plan caractéristique s'appuie sur la surface des deux sphères qui sont associées aux crêtes iliaques.

2. Procédé selon la revendication 1, **caractérisé en ce qu'**on utilise une tête à ultrasons qui effectue l'émission et la réception d'un rayonnement ultrasonore par l'intermédiaire d'un grand nombre de dispositifs d'émission et respectivement de réception, qui sont agencés en une rangée et diffusent le rayonnement ultrasonore dans un plan commun.

3. Dispositif pour la mise en oeuvre du procédé selon l'une des revendications 1 ou 2, comprenant une tête à ultrasons (10) pour l'émission et la réception de rayonnement ultrasonore et un dispositif de visualisation (12) pour la représentation d'une image correspondant à la durée de propagation d'un rayonnement ultrasonore émis et ayant été réfléchi sur une structure marquante (14, 16, 18) du corps (8), des éléments de marquage (2, 7) pour la tête à ultrasons (10) et le corps (8) étant prévus, qui font partie d'un système de navigation (3), et une unité informatique de traitement de données (6) qui est programmée de manière à déterminer, à partir de la position relative et de l'orientation relative de la tête à ultrasons (10) par rapport au corps (8) et à partir de la direction de rayonnement de la tête à ultrasons (10) et de la durée de propagation mesurée du rayonnement ultrasonore, la position et l'orientation d'une structure marquante (14, 16, 18) représentée sur le dispositif de visualisation (12) par rapport au corps, et
**caractérisé en ce que** l'unité informatique de traitement de données (6) est programmée de manière à déterminer, à partir de la position d'une structure géométrique prédéterminée, qui a été amenée le mieux possible en coïncidence avec les crêtes iliaques d'un os de la hanche sur le dispositif de visualisation, des données de structure de la structure marquante du corps, la structure géométrique prédéterminée englobant deux cercles, et l'unité informatique de traitement de données (6) déterminant en guise de données de structure de la structure géométrique prédéterminée, des sphères qui présentent les cercles de la structure prédéterminée en tant que grande cercle, et sélectionnant un plan caractéristique de l'os de la hanche de façon telle qu'un point caractéristique de la symphyse y soit situé et que le plan caractéristique s'appuie sur la surface des deux sphères qui sont associées aux crêtes iliaques.

4. Dispositif selon la revendication 3, **caractérisé en ce que** la tête à ultrasons (10) effectue l'émission et la réception d'un rayonnement ultrasonore par l'intermédiaire d'un grand nombre de dispositifs d'émission et respectivement de réception, qui sont agencés en une rangée et diffusent le rayonnement ultrasonore dans un plan commun.
